(19) Europäisches Patentamt
European Patent Office
Office européen des brevets

(11) EP 4 544 984 A1

(12) EUROPEAN PATENT APPLICATION
published in accordance with Art. 153(4) EPC

(43) Date of publication:
30.04.2025 Bulletin 2025/18

(21) Application number: 23826804.9

(22) Date of filing: 27.04.2023

(51) International Patent Classification (IPC):
*A61B 1/07* (2006.01)   *A61B 1/06* (2006.01)

(52) Cooperative Patent Classification (CPC):
A61B 1/06; A61B 1/07

(86) International application number:
PCT/JP2023/016623

(87) International publication number:
WO 2023/248615 (28.12.2023 Gazette 2023/52)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 24.06.2022 JP 2022101896

(71) Applicant: Hoya Corporation
Tokyo 160-8347 (JP)

(72) Inventor: TAKAKUBO Yutaka
Tokyo 160-8347 (JP)

(74) Representative: Schaumburg und Partner
Patentanwälte mbB
Mauerkircherstraße 31
81679 München (DE)

(54) ILLUMINATION DEVICE FOR ENDOSCOPE, AND ENDOSCOPE

(57) An endoscope illumination device includes: a light source unit that includes a light emitting element that emits excitation light and a phosphor that is excited by the excitation light to emit fluorescence, and emits illumination light in which the excitation light and the fluorescence are mixed; and a light distribution lens having an emission surface that emits the illumination light incident from the light source unit. The emission surface includes a first emission surface and a second emission surface formed in different regions in the emission surface. In the endoscope illumination device, the thickness of the light emitting element and the thickness of the phosphor are defined such that the excitation light and the fluorescence emitted from the light source unit at the same angle and incident on the light distribution lens are emitted in substantially the same direction from the first emission surface or the second emission surface, respectively.

FIG. 5

EP 4 544 984 A1

## Description

Technical Field

The present invention relates to an endoscope illumination device and an endoscope.

Background Art

Endoscopes without light guide fibers are known. For example, Patent Literature 1 describes a specific configuration of such a type of endoscope.

The endoscope described in Patent Literature 1 includes a light source chip that illuminates the inside of a body cavity at a distal end portion of an insertion tube. Since it is not necessary to guide the illumination light supplied from the light source device to the distal end portion of the insertion tube, the light guide fiber is unnecessary.

A light source chip included in this type of endoscope includes, for example, a blue light emitting diode (LED) and a phosphor (such as a phosphor that is excited by blue light and emits yellow light). The light source chip emits white light created by mixing blue light and yellow light.

Citation List

Patent Literature

Patent Literature 1: JP 2019-136248 A

Summary of Invention

Technical Problem

For example, a large intestine endoscope is required to have performance capable of capturing a reverse side of large intestine folds. For this purpose, a light distribution lens capable of distributing illumination light from the light source chip in a wide range is required. However, when a light distribution lens is designed so that illumination light including a plurality of types of light (for example, blue light and yellow light) having different wavelengths emitted from a light source chip can be distributed in a wide range, color unevenness of the illumination light increases, and color unevenness of a captured image increases.

The present invention has been made in view of the above circumstances, and an object thereof is to provide an endoscope illumination device enabling suppression of color unevenness of illumination light, and an endoscope including such an endoscope illumination device.

Solution to Problem

An endoscope illumination device according to an embodiment of the present invention includes: a light source unit that includes a light emitting element that emits excitation light and a phosphor that is excited by the excitation light to emit fluorescence, and emits illumination light in which the excitation light and the fluorescence are mixed; and a light distribution lens having an emission surface that emits the illumination light incident from the light source unit. The emission surface includes a first emission surface and a second emission surface formed in different regions in the emission surface. In the endoscope illumination device, the thickness of the light emitting element and the thickness of the phosphor are defined such that the excitation light and the fluorescence emitted from the light source unit at the same angle and incident on the light distribution lens are emitted in substantially the same direction from the first emission surface or the second emission surface, respectively.

The endoscope illumination device according to an embodiment of the present invention may include a support having an installation surface on which the light source unit is installed. In this case, the light source unit is configured such that the phosphor covers the light emitting element. A thickness of the light emitting element is a thickness from a first surface of the light emitting element, the first surface being in contact with the installation surface, to a second surface of the light emitting element, the second surface being located on a side opposite to the first surface of the light emitting element and facing the light distribution lens. A thickness of the phosphor is a thickness from a first surface of the phosphor, the first surface being in contact with the installation surface, to a second surface of the phosphor, the second surface being located on a side opposite to the first surface of the phosphor and facing the light distribution lens. In the endoscope illumination device according to an embodiment of the present invention, when a thickness of the light emitting element is D1 and a thickness of the phosphor is D2, the following formula is satisfied:

$$D2/D1 \leq 3.5.$$

In an embodiment of the present invention, when a direction in which the support and the light source unit are arranged in order is a front direction, and a direction orthogonal to the front direction is a lateral direction, the first emission surface is positioned, for example, in the front direction of the light source unit, and the second emission surface is positioned, for example, in the lateral direction of the light source unit. The emission surface includes, for example, a connection surface connecting the first emission surface and the second emission surface.

The connection surface connecting the first emission surface and the second emission surface is formed of, for example, a curved surface.

In an embodiment of the present invention, when a direction opposite to the front direction is a rear direction, the second emission surface may be formed to extend to the rear direction of the light source unit.

In an embodiment of the present invention, the light distribution lens, for example, includes an incident surface facing the second surface, emits the illumination light incident on the incident surface from the emission surface, and includes a concave curved surface formed on the incident surface for increasing a light distribution angle of the illumination light.

The concave curved surface formed on the incident surface of the light distribution lens includes, for example, a plurality of curved surfaces having different curvature radii.

When a distance in the front direction between a curvature center of a first curved surface formed closest to the connection surface among the plurality of curved surfaces and a center position of the light source unit is Z, the endoscope illumination device according to an embodiment of the present invention may satisfy the following formula:

$$Z/D2 \geq 0.1.$$

In the foregoing, the first curved surface is formed at a position intersecting a line segment connecting a center position of the curved surface forming the connection surface and the center position of the light source unit.

The light distribution lens is formed in an annular shape, and when a curvature radius of the curved surface forming the connection surface is Re and a curvature radius of the first curved surface is R1, in at least a part of an angle range of the light distribution lens formed in the annular shape, the endoscope illumination device according to an embodiment of the present invention may satisfy the following formula:

$$Re/R1 \leq 1.6.$$

For example, in the at least a part of the angle range, the curved surface forming the connection surface is positioned in the front direction of the light source unit.

In an embodiment of the present invention, the light distribution angle of the illumination light is, for example, 180° or greater.

The illumination light emitted from the light source unit is, for example, white light.

An endoscope according to an embodiment of the present invention includes an insertion tube, an imaging unit provided at a distal end portion of the insertion tube, and the endoscope illumination device described above provided around the imaging unit. The light distribution lens is formed in an annular shape so as to surround the imaging unit. A plurality of the light source units are arranged around the imaging unit at intervals.

Advantageous Effects of Invention

According to an embodiment of the present invention, an endoscope illumination device enabling suppression of color unevenness of illumination light and an endoscope including such an endoscope illumination device are provided.

Brief Description of Drawings

Fig. 1 is an external view of an endoscope according to an embodiment of the present invention.
Fig. 2A is an external perspective view of a distal end portion of the endoscope according to an embodiment of the present invention.
Fig. 2B is an arrow view corresponding to the arrow B in Fig. 2A.
Fig. 3 is a diagram showing an internal structure of a distal end portion of an endoscope according to an embodiment of the present invention.

Fig. 4 is a schematic internal structural view of a distal end portion of the endoscope for explaining each element of the endoscope illumination device according to an embodiment of the present invention.

Fig. 5 is an enlarged view showing a part of Fig. 4 in an enlarged manner.

Fig. 6 is a diagram illustrating an illumination model for quantifying color unevenness of illumination light.

Fig. 7 is a diagram showing a relationship between a light distribution angle and an index value.

Fig. 8A is a diagram showing a cross section of a light distribution cap and a light source chip included in a cross section taken along line Y1-Y1 in Fig. 2B.

Fig. 8B is a diagram showing a cross section of a light distribution cap and a light source chip included in a cross section taken along line X1-X1 in Fig. 2B.

Fig. 9 is a graph showing a relationship between a color unevenness evaluation value and Formula (1) in Numerical Value Examples 1 to 4.

Fig. 10 is a graph showing a relationship between a color unevenness evaluation value and Formula (2) in Numerical Value Examples 5 to 44.

Fig. 11 is a graph showing a relationship between a color unevenness evaluation value and Formula (3) in Numerical Value Examples 45 to 56.

Fig. 12 is a diagram showing light distribution characteristics of a blue LED and a yellow phosphor.

Fig. 13A is a diagram showing light beam angles of blue light and yellow light at the time of emission from the light distribution cap.

Fig. 13B is a diagram showing light beam angles of blue light and yellow light at the time of emission from the light distribution cap.

Fig. 13C is a diagram showing light beam angles of blue light and yellow light at the time of emission from the light distribution cap.

Fig. 14 is a diagram showing color unevenness of the illumination light.

Description of Embodiments

Hereinafter, an endoscope illumination device and an endoscope according to an embodiment of the present invention will be described with reference to the drawings.

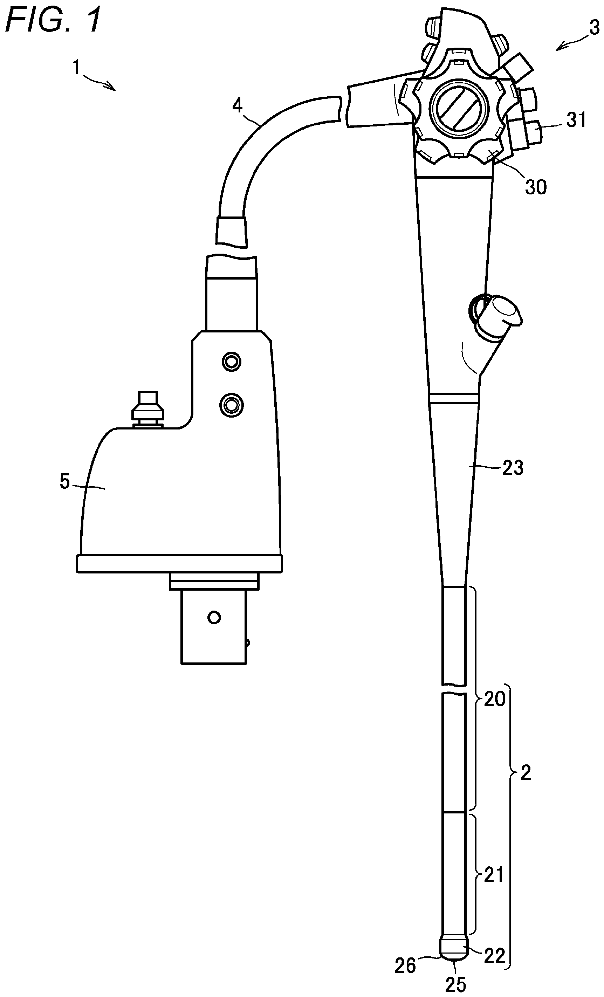

Fig. 1 is an external view of an endoscope 1 according to an embodiment of the present invention. As illustrated in FIG. 1, the endoscope 1 includes an insertion tube 2, an operation unit 3, a universal tube 4, and a connector unit 5.

The insertion tube 2 is a portion to be inserted into the body cavity, and includes a soft portion 20, a bending section 21, and a distal end portion 22. The distal end portion 22 is connected to the distal end of the soft portion 20 via the bending section 21. The base end of the soft portion 20 is connected to the operation unit 3 via a cylindrical connection portion 23.

The universal tube 4 is formed such that one end thereof is connected to the operation unit 3 and extends in a direction different from that of the insertion tube 2. The connector unit 5 is connected to the other end of the universal tube 4.

The operation unit 3 gripped by the operator is provided with operation members for performing various operations. The operation unit 3 includes a bending operation knob 30, a plurality of operation buttons 31, and the like as the operation members.

The bending operation knob 30 is connected to the bending section 21 by a wire (not illustrated) passed through the connection portion 23 and the soft portion 20. The bending section 21 is bent in two directions orthogonal to each other in the axial cross section by the operation on the bending operation knob 30. As a result, the orientation of the distal end portion 22 inserted into the body cavity is changed.

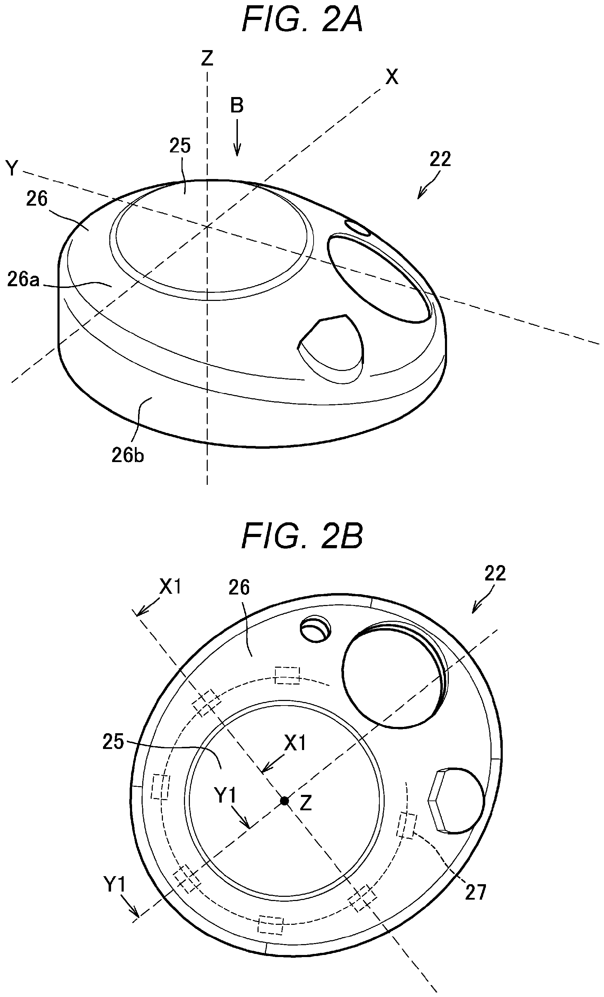

Fig. 2A is an external perspective view of the distal end portion 22. Fig. 2B is an arrow view corresponding to the arrow B in Fig. 2A. As illustrated in Figs. 2A and 2B, the optical axis direction of the objective lens 25 is defined as a Z direction, and two directions orthogonal to the Z direction and orthogonal to each other are defined as X and Y directions, respectively.

As illustrated in Figs. 2A and 2B, the distal end portion 22 is formed in an elliptical shape, and the distal end protrudes in a substantially conical shape.

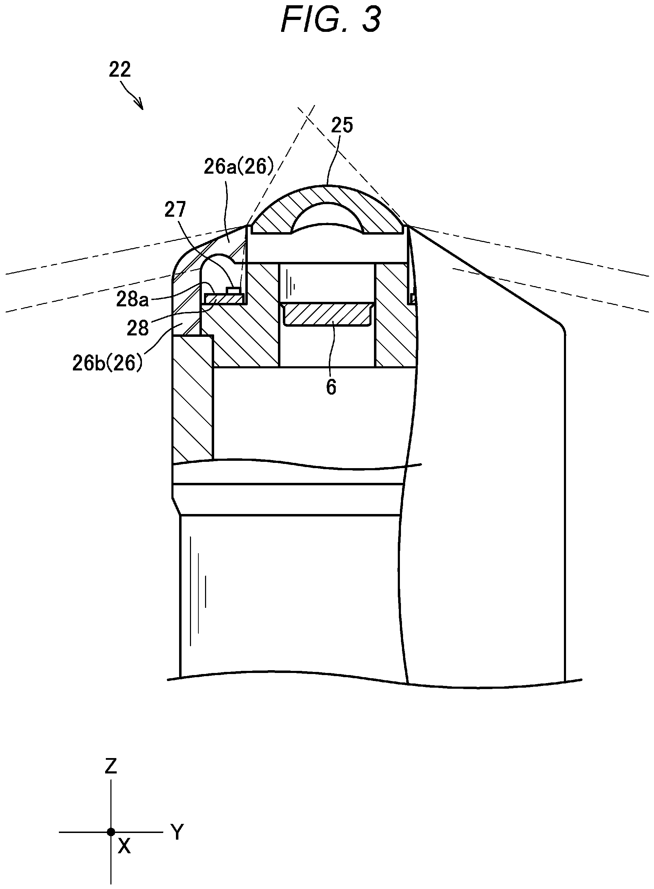

Fig. 3 is a diagram illustrating an internal structure of the distal end portion 22. The cross section illustrated in Fig. 3 is a YZ cross section of the distal end portion 22.

The distal end portion 22 is provided with an imaging unit that captures the inside of the body cavity. The imaging unit includes an image sensor 6 and an objective lens 25.

The image sensor 6 is, for example, a complementary metal oxide semiconductor (CMOS) image sensor, a charge coupled device (CCD), or the like. The objective lens 25 is a wide-angle lens.

The imaging unit including the image sensor 6 and the objective lens 25 is configured to be able to perform imaging at a viewing angle of 180° ($\pm$ 90°) or greater. In Fig. 3, a two-dot chain line indicates an imaging field of view of the imaging unit.

The endoscope illumination device according to the present embodiment is provided around the imaging unit and includes a light distribution cap 26 and a light source chip 27.

The light distribution cap 26 is an example of a light distribution lens having an emission surface that emits illumination light incident from the light source unit. The light distribution cap 26 includes a cap-shaped lens portion 26a and a cylindrical lens portion 26b.

The cap-shaped lens portion 26a is formed in a cap shape that closes the distal end of the distal end portion 22, and is formed in an annular shape so as to surround the imaging unit.

In addition, the light distribution cap 26 has a shape in which the cap-shaped lens portion 26a expands radially outward of the distal end portion 22 from the peripheral edge portion of the objective lens 25, passes through a portion formed to be a curved surface (described later), and is continuous with the cylindrical lens portion 26b forming a part of the side wall of the distal end portion 22.

An annular substrate 28 surrounding the imaging unit is provided in the distal end portion 22. A plurality of the light source chips 27 are disposed on the top surface 28a of the substrate 28 at intervals around the imaging unit. In Fig. 2B, as an example, seven light source chips 27 are disposed.

The light source chips 27 are an example of the light source unit. The substrate 28 is an example of the support having the installation surface (top surface 28a) on which the light source unit is installed.

The illumination light emitted from the light source chip 27 is emitted to the outside through the light distribution cap 26 to illuminate the imaging field of view of the imaging unit.

In order to illuminate a wide imaging field of view, the light distribution cap 26 is designed so that the illumination light from the light source chip 27 can be distributed over a wide range. In Fig. 3, a broken line indicates a light distribution range by the light distribution cap 26. The light distribution angle of the illumination light by the light distribution cap 26 is, for example, 180° ($\pm$ 90°) or greater.

The light source chip 27 includes a light emitting element that emits excitation light and a phosphor that is excited by the excitation light to emit fluorescence. The light source chip 27 is configured such that a phosphor covers the light emitting element, and emits illumination light in which excitation light and fluorescence are mixed.

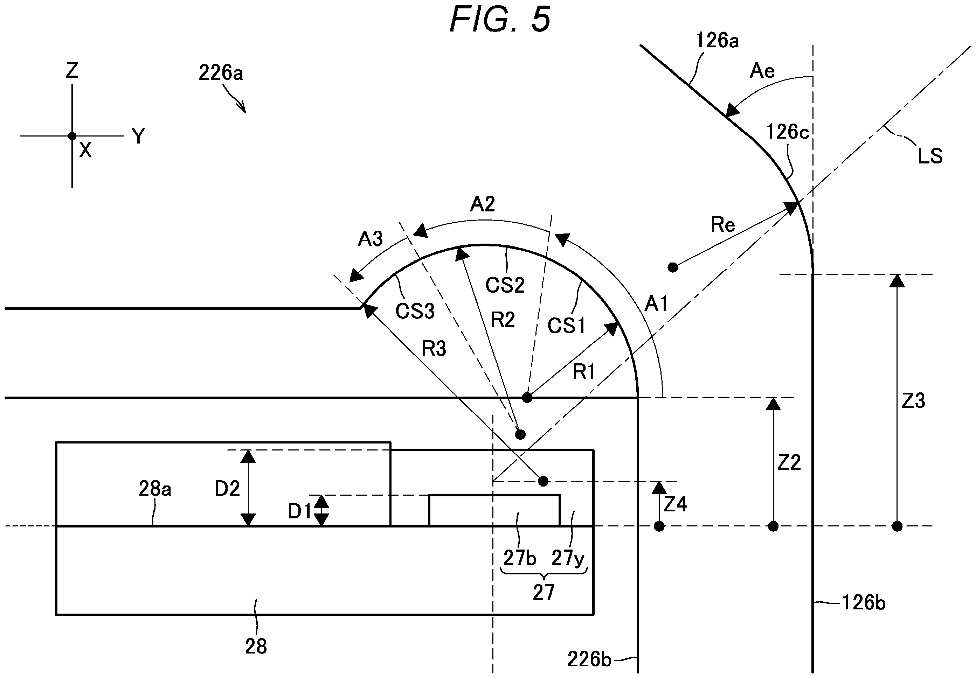

In the present embodiment, the light emitting element is a blue LED that emits blue light (see reference numeral 27b in Fig. 5 described later). The phosphor is a yellow phosphor (see reference numeral 27y in Fig. 5 described later) that is excited by blue light to emit yellow light. The light source chip 27 emits white light created by mixing the blue light and the yellow light.

The configuration of the light source chip 27 that emits white light is not limited to the above configuration. The light source chip 27 may include a blue LED, a red phosphor, and a green phosphor, or may include an ultraviolet LED, a blue phosphor, a green phosphor, and a red phosphor.

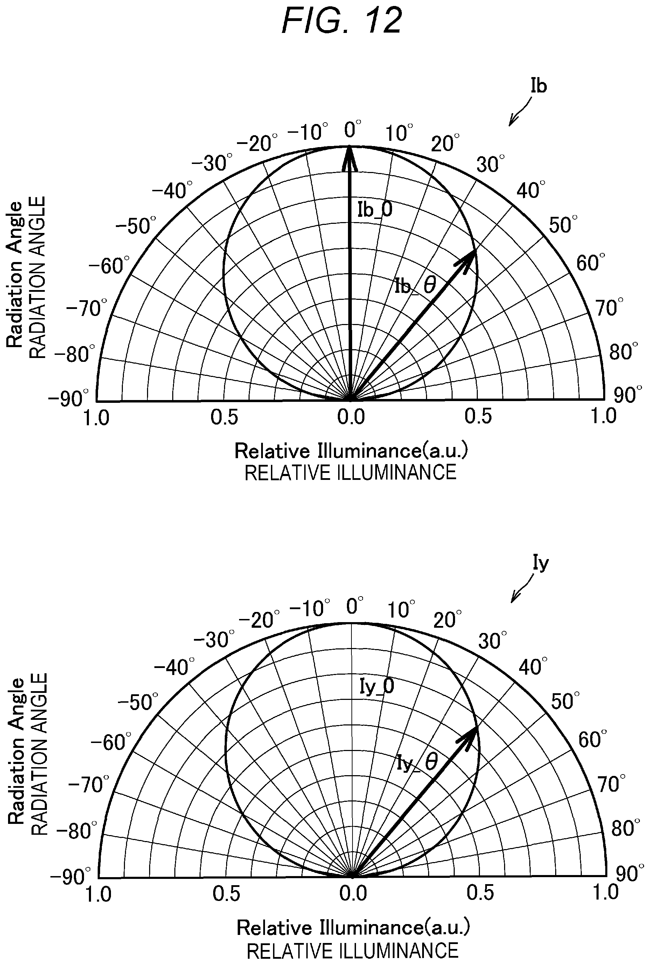

Fig. 12 is a diagram showing light distribution characteristics of the blue LED and the yellow phosphor. In Fig. 12, reference sign Ib represents the light distribution characteristics of the blue LED, and reference sign Iy represents the light distribution characteristics of the yellow phosphor.

Both the light distribution characteristics of the blue LED and the yellow phosphor are characteristics in which the intensity of light emitted from the light emitting surface at an angle θ is COSθ times the intensity of light emitted vertically, that is, Lambertian light distribution.

A case where the light source chip 27 is arranged at the center of the sphere, and the inner surface of the sphere sufficiently distant from the light source chip 27 is illuminated by the light source chip 27 will be considered hereinafter.

In this case, a point on the sphere in the direction of the light distribution angle of 0° is illuminated with light of intensity Ib_0 emitted from the blue LED at the light distribution angle of 0° and light of intensity Iy_0 emitted from the yellow phosphor at the light distribution angle of 0°. The light intensity ratio Ib_0/Iy_0 is set such that the illumination light illuminating the point on the sphere in the direction of the light distribution angle of 0° is white.

In addition, a point on the sphere in the direction of the light distribution angle θ is illuminated with light of intensity Ib_θ emitted from the blue LED at the light distribution angle θ and light of intensity Iy_θ emitted from the yellow phosphor at the light distribution angle θ. The light intensity ratio Ib_θ/Iy_θ is set such that the illumination light illuminating the point on the sphere in the direction of the light distribution angle θ is white.

That is, the light source chip 27 is set to have characteristics in which the intensity ratio Ib_0/Iy_0 and the intensity ratio Ib_θ/Iy_θ substantially coincide with each other so that a wide range can be illuminated with white light.

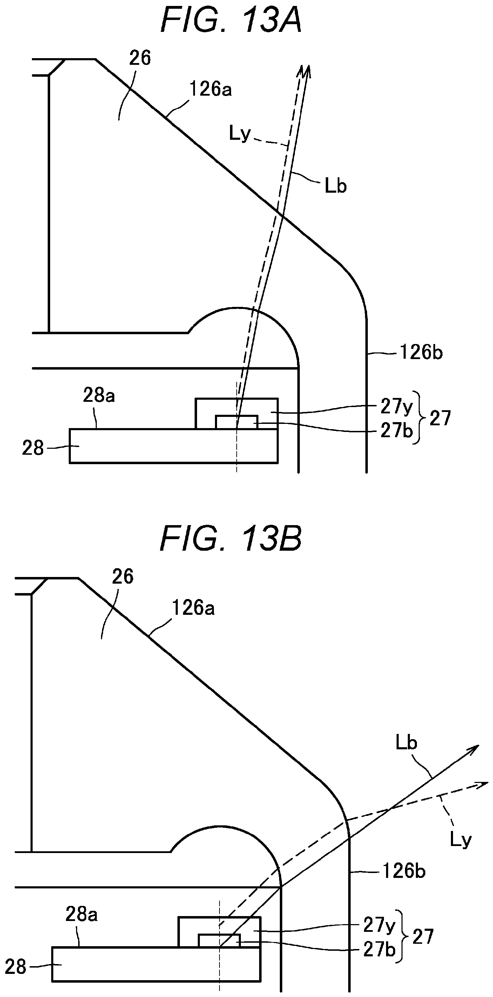

Figs. 13A to 13C are schematic diagrams showing a positional relationship between the light distribution cap 26 and the light source chip 27. As illustrated in Figs. 13A to 13C, the light source chip 27 includes the blue LED 27b and the yellow phosphor 27y. The blue light emitted from the blue LED 27b is referred to as "blue light Lb", and the yellow light emitted from the yellow phosphor 27y is referred to as "yellow light Ly".

As illustrated in Figs. 13A to 13C, the blue light Lb at each angle emitted from the blue LED 27b and the yellow light Ly at each angle emitted from the yellow phosphor 27y are incident on the light distribution cap 26 and emitted to the outside from the emission surface of the light distribution cap 26.

Fig. 13A illustrates the blue light Lb and the yellow light Ly emitted at an angle close to perpendicular to the emission surface of the blue LED 27b and the yellow phosphor 27y. Such blue light Lb and yellow light Ly pass through substantially the same position on the light distribution cap 26. Therefore, the blue light Lb and the yellow light Ly have substantially the same light beam angle when emitted from the light distribution cap 26, and illuminate substantially the same region on the surface to be irradiated. Therefore, the intensity ratio Ib_$\theta$/Iy_$\theta$ becomes a value close to the intensity ratio Ib_0/Iy_0, and the surface to be illuminated is illuminated substantially in white.

Fig. 13B illustrates the blue light Lb and the yellow light Ly emitted at an angle not close to perpendicular (for example, 45°) to the emission surface of the blue LED 27b and the yellow phosphor 27y. Such blue light Lb and yellow light Ly pass through relatively distant positions on the light distribution cap 26. Therefore, the blue light Lb and the yellow light Ly have greatly different light beam angles when emitted from the light distribution cap 26, and illuminate different regions on the surface to be irradiated. The region on the surface to be irradiated where the blue light Lb reaches is illuminated with a bluish color, and the region on the irradiation surface where the yellow light Ly reaches is illuminated with a yellowish color.

That is, in the case of Fig. 13B, since the color unevenness of the illumination light is significant, the color unevenness occurs in the captured image.

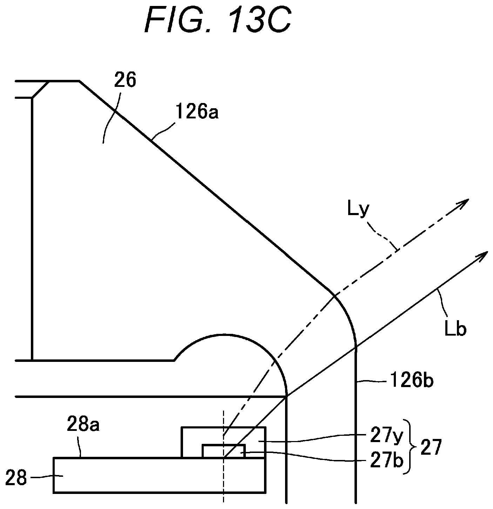

Fig. 13C illustrates the blue light Lb and the yellow light Ly (blue light Lb emitted at a light distribution angle $\theta$ and yellow light Ly emitted at a light distribution angle $\theta$') emitted at different angles with respect to the emission surfaces of the blue LED 27b and the yellow phosphor 27y.

As illustrated in Fig. 13C, among the blue light Lb and the yellow light Ly emitted at different light distribution angles, there are blue light Lb and yellow light Ly that have substantially the same light beam angle when emitted from the light distribution cap 26 and illuminate substantially the same region on the surface to be irradiated. However, the intensity ratio is Ib_$\theta$/Iy_$\theta$', which is different from Ib_0/Iy_0. Therefore, the region on the surface to be irradiated is illuminated with a bluish color or a yellowish color.

Depending on the shapes of the incident surface and the emission surface of the light distribution cap 26, a difference in light beam angle between the blue light Lb and the yellow phosphor 27y after being transmitted through the light distribution cap 26 increases to increase color unevenness, or a difference in light beam angle decreases to reduce color unevenness. In addition, as illustrated in Figs. 13A to 13C, when the incident surface and the emission surface of the light distribution cap 26 are formed into a complicated shape in order to set the light distribution angle of the illumination light to 180° or greater, the light beam angles of the blue light Lb and the yellow phosphor 27y after being transmitted through the light distribution cap 26 are changed in a complicated manner. Therefore, color unevenness of the illumination light is likely to occur.

As a result of intensive studies, the present inventor has obtained a finding that a deviation between the light emission position of the blue LED 27b and the light emission position of the yellow phosphor 27y in the height direction is one of factors that cause color unevenness in the illumination light.

Therefore, in the present embodiment, the light emission position of the blue LED 27b and the light emission position of the yellow phosphor 27y are appropriately set (in other words, the thickness of the blue LED 27b and the thickness of the yellow phosphor 27y are appropriately defined) so that color unevenness is less likely to occur in a wide range on the surface to be irradiated.

Specifically, the endoscope illumination device according to the present embodiment has a configuration in which the thickness of the blue LED 27b and the thickness of the yellow phosphor 27y are defined such that the blue light Lb and the yellow light Ly emitted from the light source chip 27 at the same angle and incident on the light distribution cap 26 are emitted in substantially the same direction from the first emission surface (as an example, the emission surface 126a of the cap-shaped lens portion 26a) or the second emission surface (as an example, the emission surface 126b of the cylindrical lens portion 26b), respectively.

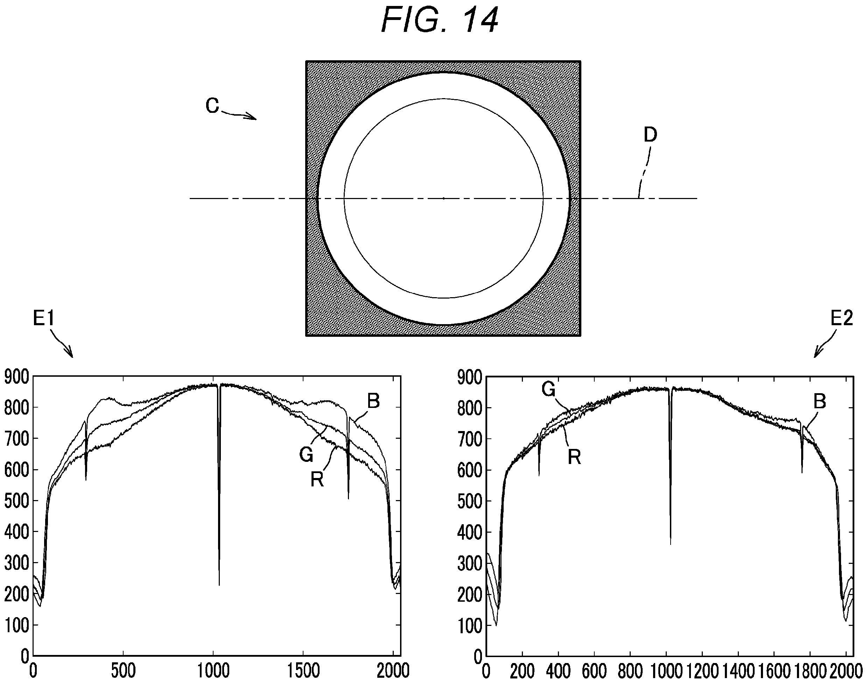

Fig. 14 is a diagram showing color unevenness of the illumination light. In Fig. 14, reference numeral C denotes an example of an image in a case where an inner surface of a sphere away from the distal end portion of the endoscope by a certain distance is captured. In Fig. 14, reference numeral E1 denotes an intensity distribution of R, G, and B on a line D in the image example C captured by a conventional endoscope. In Fig. 14, reference numeral E2 denotes an intensity distribution of R, G, and B on the line D in the image example C captured by the endoscope 1 according to the present embodiment.

As can be seen by comparing the intensity distributions E1 and E2, in the endoscope 1 according to the present embodiment, the color unevenness of the captured image is suppressed as compared with the related art.

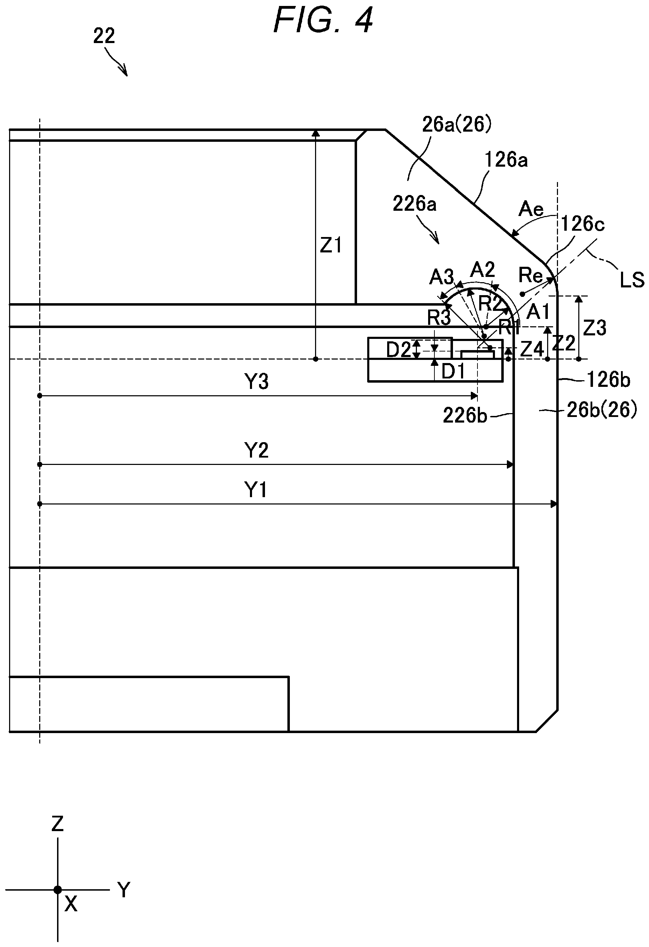

Fig. 4 is a schematic internal structural view of the distal end portion 22 for explaining each element of the endoscope illumination device according to the present embodiment. Fig. 5 is an enlarged view showing a part of Fig. 4 in an enlarged manner.

Reference numeral D1 indicates the thickness of the blue LED 27b. The thickness D1 of the blue LED 27b is a thickness from the bottom surface (an example of the first surface of the light emitting element) of the blue LED 27b in contact with the top surface 28a of the substrate 28 to the light emitting surface (an example of the second surface of the light emitting element) of the blue LED 27b located on the opposite side of the bottom surface and facing the light distribution cap 26.

Reference numeral D2 denotes a thickness of the yellow phosphor 27y. The thickness D2 of the yellow phosphor 27y is a thickness from the bottom surface (an example of the first surface of the phosphor) of the yellow phosphor 27y in contact with the top surface 28a of the substrate 28 to the light emitting surface (an example of the second surface of the phosphor) of the yellow phosphor 27y located on the opposite side of the bottom surface and facing the light distribution cap 26.

The inner surface of the light distribution cap 26 is an incident surface on which the blue light Lb and the yellow light Ly are incident. As illustrated in Figs. 4 and 5, the incident surface of the light distribution cap 26 includes a concave curved surface portion 226a and an inner peripheral portion 226b parallel to the emission surface 126b of the cylindrical lens portion 26b in the cross-sectional view.

The incident surface (at least curved surface portion 226a) of light distribution cap 26 is positioned to face the light emitting surfaces of the blue LED 27b and the yellow phosphor 27y. The curved surface portion 226a is formed in a concave shape in order to increase the light distribution angle of the illumination light.

The curved surface portion 226a includes a plurality of curved surfaces having different curvature radii. The curved surface portion 226a includes two curved surfaces in a part of the angle range of the light distribution cap 26 formed in an annular shape, and includes three curved surfaces in the other angle range.

As illustrated in Figs. 4 and 5, the first curved surface connected to the inner peripheral portion 226b is formed with a curvature radius R1 and is formed in an angular range indicated by reference sign A1. The second curved surface connected to the first curved surface is formed with a curvature radius R2 and is formed in an angular range indicated by reference sign A2. The third curved surface connected to the second curved surface is formed with a curvature radius R3 and is formed in an angular range indicated by reference sign A3. As illustrated in Fig. 5, for convenience, the first curved surface, the second curved surface, and the third curved surface are respectively denoted by reference signs CS1, CS2, and CS3.

In Figs. 4 and 5, the curved surface portion 226a has a shape including three curved surfaces. When the curved surface portion 226a has a shape including two curved surfaces, the first curved surface CS1 is formed in the angular range A1, and the second curved surface CS2 is formed in the angular ranges A2 and A3.

The outer surface of the light distribution cap 26 is an emission surface from which the blue light Lb and the yellow light Ly incident on the incident surface are emitted. The light distribution cap 26 includes a first emission surface and a second emission surface formed in different regions in the emission surface. More specifically, the emission surface of the light distribution cap 26 includes an emission surface 126a of the cap-shaped lens portion 26a, an emission surface 126b of the cylindrical lens portion 26b, and an emission surface 126c (an example of a connection surface) that connects the emission surface 126a and the emission surface 126b.

The emission surface 126b of the cylindrical lens portion 26b is formed to extend in the Z direction. On the other hand, the emission surface 126a of the cap-shaped lens portion 26a is formed at an angle with respect to the Z direction. In Figs. 4 and 5, reference sign Ae denotes an angle formed by the emission surface 126a and the emission surface 126b.

The emission surface 126c is formed of a curved surface having a curvature radius Re.

When a direction in which the substrate 28 and the light source chip 27 are arranged in order is a front direction (in Fig. 4, the upward direction and the Z direction), and a direction orthogonal to the front direction is a lateral direction (in Fig. 4, the lateral direction and the Y direction), the emission surface 126a as an example of the first emission surface is located in the front direction of the light source chip 27, and the emission surface 126b as an example of a second emission surface is located in the lateral direction of the light source chip 27.

When a direction opposite to the front direction is a rear direction (in Fig. 4, the downward direction and the Z direction), the emission surface 126b, which is an example of the second emission surface, is formed to extend to the rear direction of the light source chip 27.

In Figs. 4 and 5, a line segment LS connects the center position of the emission surface 126c and the center position of the light source chip 27. The first curved surface CS1 is formed at a position intersecting the line segment LS. In addition, the first curved surface CS1 is formed closest to the emission surface 126c which is an example of the connection surface among the plurality of curved surfaces forming the curved surface portion 226a.

In Figs. 4 and 5, a distance Y1 indicates a distance in the Y direction from the central axis of the light distribution cap 26 (coinciding with the optical axis of the objective lens 25) to the emission surface 126b of the cylindrical lens portion 26b.

In Figs. 4 and 5, a distance Y2 indicates a distance in the Y direction from the central axis of the light distribution cap 26 to the inner peripheral portion 226b of the light distribution cap 26.

In Figs. 4 and 5, a distance Y3 indicates a distance in the Y direction from the center axis of the light distribution cap 26 to the center position of the light source chip 27.

In Figs. 4 and 5, a distance Z1 indicates a distance in the Z direction from the bottom surface (in other words, the bottom surface of the blue LED 27b, the bottom surface of the yellow phosphor 27y, or the top surface 28a of the substrate 28) of light source chip 27 to the distal end surface of light distribution cap 26.

In Figs. 4 and 5, a distance Z2 indicates a distance in the Z direction from the bottom surface of light source chip 27 to the boundary position between curved surface portion 226a and inner peripheral portion 226b.

In Figs. 4 and 5, a distance Z3 indicates a distance in the Z direction from the bottom surface of the light source chip 27 to a boundary position between the emission surface 126b and the emission surface 126c.

In Figs. 4 and 5, distance Z4 indicates a distance in the Z direction from the bottom surface of light source chip 27 to the center position of light source chip 27.

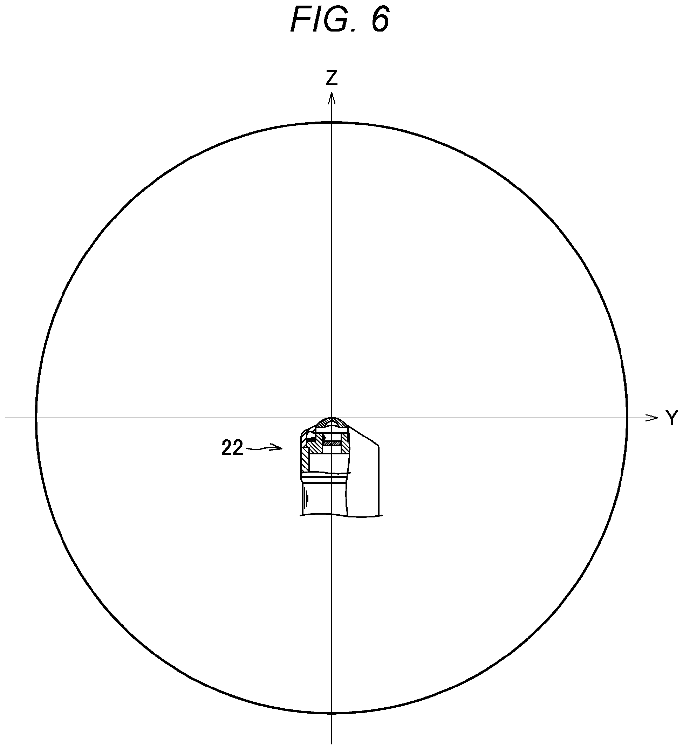

Quantification of color unevenness of illumination light will be described hereinafter. Fig. 6 illustrates an illumination model for quantifying color unevenness of illumination light. In the illumination model illustrated in Fig. 6, the distal end portion 22 is arranged at the center of the sphere, and the inner surface of the sphere sufficiently distant from the distal end portion 22 is illuminated with the illumination light. In this illumination model, the Z-axis direction is set to 0°, and the Y-axis direction is set to 90°.

When the light amount of the blue light Lb incident on a certain angular position on the spherical surface is B, and the light amount of the yellow light Ly incident on the same angular position is Y, the light amount B/light amount Y represents the light amount ratio between the blue light Lb and the yellow light Ly at the angular position. Light amount B/light amount Y is an index value indicating the hue of the illumination light obtained by mixing the blue light Lb and the yellow light Ly having different wavelengths. Hereinafter, this index value is referred to as an "index value I".

The index value I changes depending on the angular position. This indicates that color unevenness of the illumination light occurs on the surface to be irradiated. As the index value I (that is, light amount B/light amount Y) increases, the illumination light becomes bluish. As the index value I decreases, the illumination light becomes yellowish.

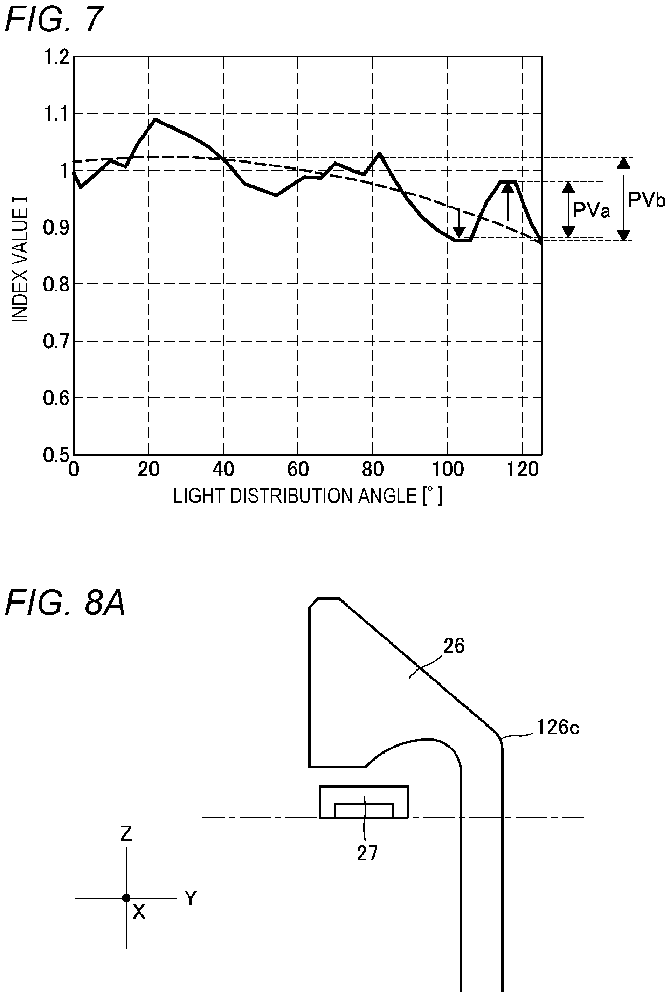

Fig. 7 illustrates a relationship between the angular position (in other words, the light distribution angle (°)) and the index value I. In Fig. 7, the vertical axis represents the index value I, and the horizontal axis represents the light distribution angle (°). In the example of Fig. 7, the index value I of the light distribution angle of 0° (position on the Z axis) is normalized to 1. The illumination optical system constituted by the endoscope illumination device is substantially rotationally symmetric with respect to the axis of the light distribution angle of 0°. Therefore, in Fig. 7, only the index value I of the positive light distribution angle is illustrated.

In Fig. 7, a solid line connects the light distribution angle of 0° to 124° at a pitch of 4°, and a broken line approximates the solid line by a quadratic expression.

The PV (Peak-to-valley) of the deviation of the solid line with respect to the approximate line (broken line) is defined as PVa, and the PV of the approximate line is defined as PVb. In the present embodiment, a value obtained by adding PVa to a value obtained by dividing PVb by 2 (that is, PVa + (PVb/2)) is defined as a "color unevenness evaluation value" for quantitatively evaluating color unevenness.

As the color unevenness evaluation value is smaller, the color difference of the illumination light over the entire light distribution angle, that is, the color unevenness, is smaller. The human eye has higher tolerance for the color unevenness that changes gently than the color unevenness that changes rapidly (in other words, it is difficult to perceive color unevenness if the change is gentle). In accordance with the characteristics of the human eye, in the above formula for defining the color unevenness evaluation value, the value PVb corresponding to the gentle color unevenness is divided by 2, while the value PVa corresponding to the sudden color unevenness is not divided.

In the present embodiment, the allowable color unevenness evaluation value is 0.5 or less. A color unevenness evaluation value of 0.4 or less is more desirable. A color unevenness evaluation value of 0.3 or less is still more desirable.

As described above, in order to suppress the color unevenness of the illumination light over a wide range in the surface to be irradiated, the present embodiment has a configuration in which the thickness of the blue LED 27b and the thickness of the yellow phosphor 27y are defined such that the blue light Lb and the yellow light Ly emitted from the light source chip 27 at the same angle and incident on the light distribution cap 26 are emitted in substantially the same direction from the first emission surface (as an example, the emission surface 126a of the cap-shaped lens portion 26a) or the second emission surface (as an example, the emission surface 126b of the cylindrical lens portion 26b), respectively.

As a more specific example, the thickness D1 of the blue LED 27b and the thickness D2 of the yellow phosphor 27y satisfy the following Formula (1):

$$D2/D1 \leq 3.5 \dots (1).$$

When the blue LED 27b and the yellow phosphor 27y have the same thickness, color unevenness of illumination light can be suppressed. However, in order to cause the excitation light from the blue LED 27b to efficiently enter the yellow phosphor 27y, it is necessary to configure the light source chip 27 such that the yellow phosphor 27y covers the blue LED 27b. Therefore, it is difficult to make the blue LED 27b and the yellow phosphor 27y have the same thickness.

On the other hand, since the blue LED 27b and the yellow phosphor 27y also have a certain size in the width direction orthogonal to the thickness direction and the depth direction (for example, in Fig. 4, X and Y directions orthogonal to the Z direction), the color unevenness on the surface to be irradiated is also uniformized to some extent.

In consideration of the foregoing, the above Formula (1) is defined. When the above Formula (1) is satisfied, the color unevenness evaluation value is easily suppressed to 0.5 or less, and the color unevenness of the illumination light is easily suppressed favorably (see Numerical Value Examples 1 to 56 described later).

Here, the degree of color unevenness of the illumination light also varies depending on the inner surface (incident surface) shape and the outer surface (emission surface) shape of the light distribution cap 26.

For example, as described above, the emission surface 126c of the light distribution cap 26 is formed as a curved surface. The emission surface 126c connects the emission surface 126a and the emission surface 126b of the light distribution cap 26, and thus does not form a gentle curved surface. When the curvature radius is smaller (when the curved surface is steeper), the refraction angle is more likely to change depending on the light passing position. Therefore, the emission surface 126c is likely to cause color unevenness of the illumination light. However, in the present embodiment, by satisfying the above Formula (1), the color unevenness of the illumination light can be favorably suppressed even if the emission surface of the light distribution cap 26 has a shape having a local curved surface (emission surface 126c).

As described above, the emission surface 126b, which is an example of the second emission surface, is formed to extend to the rear direction of the light source chip 27. When the emission surface 126b is formed to extend to the rear direction of the light source chip 27, the emission range of the illumination light from the light distribution cap 26 expands. Therefore, such a configuration leads to expansion of the light distribution angle of the illumination light, and can also be a factor that causes color unevenness of the illumination light. However, in the present embodiment, by satisfying the above Formula (1), even when the emission surface 126b is formed to extend to the rear direction of the light source chip 27, the color unevenness of the illumination light can be favorably suppressed.

As described above, the incident surface of light distribution cap 26 includes curved surface 226a formed in the concave shape. When the curvature radius of the curved surface portion 226a is reduced (when the curved surface is steeper) in order to increase the light distribution angle of the illumination light, the refraction angle is more likely to change depending on the light passing position. Therefore, the curved surface portion 226a is likely to cause color unevenness of the illumination light. However, in the present embodiment, by satisfying the above Formula (1), the color unevenness of the illumination light is likely to be suppressed even when the curvature radius of the curved surface portion 226a is small.

As described above, the curved surface portion 226a includes a plurality of curved surfaces having different curvature radii. By forming the curved surface portion 226a with a plurality of curved surfaces having different curvature radii, it is possible to suppress color unevenness of the illumination light while expanding the light distribution angle of the illumination light.

When a distance (more specifically, a distance obtained by subtracting the distance Z4 from the distance Z2) in the Z direction between the center of curvature of the first curved surface CS1 and the center position of the light source chip 27 is Z, the endoscope illumination device according to the present embodiment satisfies the following Formula (2):

$$Z/D2 \geq 0.1 \ \ldots \ (2).$$

The distance Z takes a negative value when the distance Z4 is greater than the distance Z2.

When the above Formula (2) is satisfied, the color unevenness evaluation value is easily suppressed to 0.4 or less, and the color unevenness of the illumination light is easily suppressed favorably (see Numerical Value Examples 5 to 44 described later).

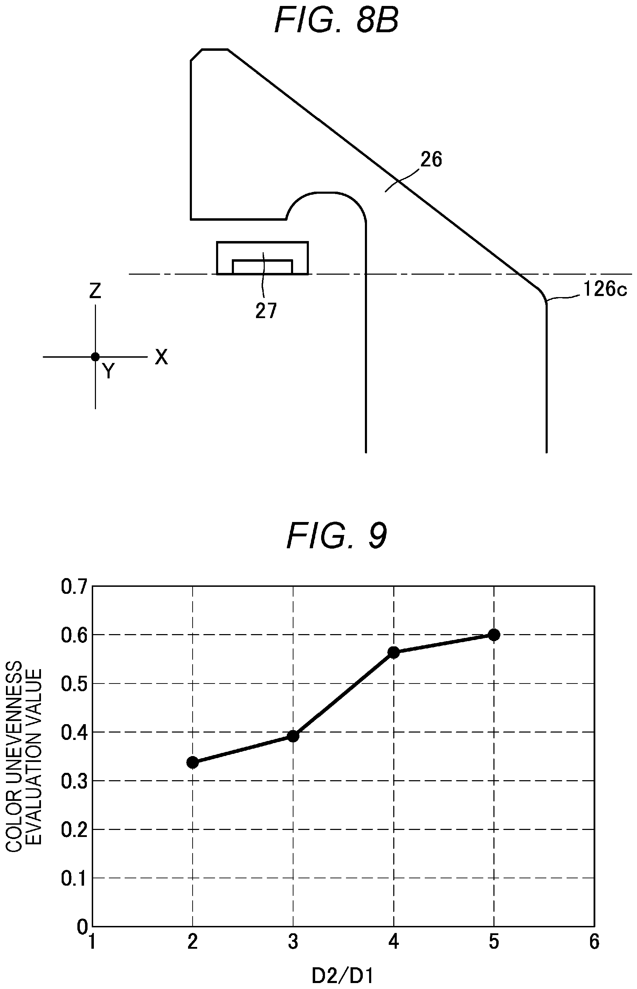

Fig. 8A shows a cross section of a light distribution cap 26 and a light source chip 27 included in a cross section taken along line Y1-Y1 in Fig. 2B. Fig. 8B shows a cross section of a light distribution cap 26 and a light source chip 27 included in a cross section taken along line X1-X1 in Fig. 2B.

In the light distribution cap 26 formed in the annular shape, the emission surface 126c is positioned in the front direction of the light source chip 27 in a partial angular range (see Fig. 8A), and the emission surface 126c is positioned in the rear direction of the light source chip 27 in the other angular range (see Fig. 8B).

In an angular range in which the emission surface 126c is located in the front direction of the light source chip 27, the endoscope illumination device according to the present embodiment satisfies the following Formula (3):

$$Re/R1 \leq 1.6 \ \ldots \ (3).$$

When the above Formula (3) is satisfied, the color unevenness evaluation value is easily suppressed to 0.3 or less, and the color unevenness of the illumination light is easily suppressed favorably (see Numerical Value Examples 45 to 56 described later).

Next, specific Numerical Value Examples will be shown.

Table 1 shows parameter values of the endoscope illumination device according to Numerical Value Examples 1 to 4.

(Table 1)

[Table 1]

| NUMERICAL VALUE EXAMPLES | | | | |
|---|---|---|---|---|
| EXAMPLE | 1 | 2 | 3 | 4 |
| Y1 [mm] | 2.6 | 2.6 | 2.6 | 2.6 |
| Y2 [mm] | 2.4 | 2.4 | 2.4 | 2.4 |
| Y3 [mm] | 2.2 | 2.2 | 2.2 | 2.2 |
| Z1 [mm] | 2 | 2 | 2 | 2 |
| Z2 [mm] | 0.6 | 0.6 | 0.6 | 0.6 |
| Z3 [mm] | 0.6 | 0.6 | 0.6 | 0.6 |
| Z4 [mm] | 0.25 | 0.20 | 0.15 | 0.10 |
| R1 [mm] | 0.2 | 0.2 | 0.2 | 0.2 |
| R2 [mm] | 0.8 | 0.8 | 0.8 | 0.8 |
| R3 [mm] | - | - | - | - |
| Re [mm] | 0.2 | 0.2 | 0.2 | 0.2 |
| A1 [deg] | 60 | 60 | 60 | 60 |
| A2 [deg] | 80 | 80 | 80 | 80 |
| A3 [deg] | - | - | - | - |
| Ae [deg] | 50 | 50 | 50 | 50 |
| D1 [mm] | 0.1 | 0.1 | 0.1 | 0.1 |
| D1 [mm] | 0.5 | 0.4 | 0.3 | 0.2 |
| D2/D1 | 5 | 4 | 3 | 2 |
| COLOR UNEVENNESS EVALUATION VALUE | 0.60 | 0.56 | 0.39 | 0.34 |

Fig. 9 is a graph showing a relationship between a color unevenness evaluation value and Formula (1) in Numerical Value Examples 1 to 4. In Fig. 9, the vertical axis represents the color unevenness evaluation value, and the horizontal axis represents the value of D2/D1.

As shown in Table 1, in Numerical Value Examples 1 to 2, the value of D2/D1 exceeds 3.5. As a result, the color unevenness evaluation value exceeds 0.5. On the other hand, in Numerical Value Examples 3 to 4, the value of D2/D1 is 3.5 or less. As a result, the color unevenness evaluation value is 0.5 or less.

As described above, in Numerical Value Examples 3 to 4, it can be seen that the color unevenness of the illumination light can be favorably suppressed by appropriately defining the thickness D1 of the blue LED 27b and the thickness D2 of the yellow phosphor 27y.

Tables 2A to 2D show parameter values of the endoscope illumination device according to Numerical Value Examples 5 to 44. In any of Numerical Value Examples 5 to 44, the value of D2/D1 is 3.0.

(Table 2A)

[Table 2A]

| NUMERICAL VALUE EXAMPLE | | | | | | | | | | |
|---|---|---|---|---|---|---|---|---|---|---|
| EXAMPLE | 5 | 6 | 7 | 8 | 9 | 10 | 11 | 12 | 13 | 14 |
| Y1 [mm] | 2.6 | 2.6 | 2.6 | 2.6 | 2.6 | 2.6 | 2.6 | 2.6 | 2.6 | 2.6 |

(continued)

| NUMERICAL VALUE EXAMPLE | | | | | | | | | | |
|---|---|---|---|---|---|---|---|---|---|---|
| EXAMPLE | 5 | 6 | 7 | 8 | 9 | 10 | 11 | 12 | 13 | 14 |
| Y2 [mm] | 2.4 | 2.4 | 2.4 | 2.4 | 2.4 | 2.4 | 2.4 | 2.4 | 2.4 | 2.4 |
| Y3 [mm] | 2.2 | 2.2 | 2.2 | 2.2 | 2.2 | 2.2 | 2.2 | 2.2 | 2.2 | 2.2 |
| Z1 [mm] | 2 | 2 | 2 | 2 | 2 | 2 | 2 | 2 | 2 | 2 |
| Z2 [mm] | 0.28 | 0.26 | 0.24 | 0.22 | 0.2 | 0.18 | 0.16 | 0.14 | 0.12 | 0.1 |
| Z3 [mm] | 0.4 | 0.4 | 0.4 | 0.4 | 0.4 | 0.4 | 0.4 | 0.4 | 0.4 | 0.4 |
| Z4 [mm] | 0.15 | 0.15 | 0.15 | 0.15 | 0.15 | 0.15 | 0.15 | 0.15 | 0.15 | 0.15 |
| R1 [mm] | 0.5 | 0.5 | 0.5 | 0.5 | 0.5 | 0.5 | 0.5 | 0.5 | 0.5 | 0.5 |
| R2 [mm] | 0.6 | 0.6 | 0.6 | 0.6 | 0.6 | 0.6 | 0.6 | 0.6 | 0.6 | 0.6 |
| R3 [mm] | 0.7 | 0.7 | 0.7 | 0.7 | 0.7 | 0.7 | 0.7 | 0.7 | 0.7 | 0.7 |
| Re [mm] | 1 | 1 | 1 | 1 | 1 | 1 | 1 | 1 | 1 | 1 |
| A1 [deg] | 50 | 50 | 50 | 50 | 50 | 50 | 50 | 50 | 50 | 50 |
| A2 [deg] | 40 | 40 | 40 | 40 | 40 | 40 | 40 | 40 | 40 | 40 |
| A3 [deg] | 63 | 63 | 63 | 63 | 63 | 63 | 63 | 63 | 63 | 63 |
| Ae [deg] | 50 | 50 | 50 | 50 | 50 | 50 | 50 | 50 | 50 | 50 |
| D1 [mm] | 0.1 | 0.1 | 0.1 | 0.1 | 0.1 | 0.1 | 0.1 | 0.1 | 0.1 | 0.1 |
| D2 [mm] | 0.3 | 0.3 | 0.3 | 0.3 | 0.3 | 0.3 | 0.3 | 0.3 | 0.3 | 0.3 |
| Z/D2 | 0.43 | 0.37 | 0.30 | 0.23 | 0.17 | 0.10 | 0.03 | -0.03 | -0.10 | -0.17 |
| COLOR UNEVENNESS EVALUATION VALUE | 0.22 | 0.23 | 0.26 | 0.31 | 0.38 | 0.42 | 0.38 | 0.47 | 0.42 | 0.53 |

(Table 2B)

[Table 2B]

| NUMERICAL VALUE EXAMPLE | | | | | | | | | | |
|---|---|---|---|---|---|---|---|---|---|---|
| EXAMPLE | 15 | 16 | 17 | 18 | 19 | 20 | 21 | 22 | 23 | 24 |
| Y1 [mm] | 2.6 | 2.6 | 2.6 | 2.6 | 2.6 | 2.6 | 2.6 | 2.6 | 2.6 | 2.6 |
| Y2 [mm] | 2.4 | 2.4 | 2.4 | 2.4 | 2.4 | 2.4 | 2.4 | 2.4 | 2.4 | 2.4 |
| Y3 [mm] | 2.2 | 2.2 | 2.2 | 2.2 | 2.2 | 2.2 | 2.2 | 2.2 | 2.2 | 2.2 |
| Z1 [mm] | 2 | 2 | 2 | 2 | 2 | 2 | 2 | 2 | 2 | 2 |
| Z2 [mm] | 0.28 | 0.26 | 0.24 | 0.22 | 0.2 | 0.18 | 0.16 | 0.14 | 0.12 | 0.1 |
| Z3 [mm] | 0.4 | 0.4 | 0.4 | 0.4 | 0.4 | 0.4 | 0.4 | 0.4 | 0.4 | 0.4 |
| Z4 [mm] | 0.15 | 0.15 | 0.15 | 0.15 | 0.15 | 0.15 | 0.15 | 0.15 | 0.15 | 0.15 |
| R1 [mm] | 0.55 | 0.55 | 0.55 | 0.55 | 0.55 | 0.55 | 0.55 | 0.55 | 0.55 | 0.55 |
| R2 [mm] | 0.6 | 0.6 | 0.6 | 0.6 | 0.6 | 0.6 | 0.6 | 0.6 | 0.6 | 0.6 |
| R3 [mm] | 0.7 | 0.7 | 0.7 | 0.7 | 0.7 | 0.7 | 0.7 | 0.7 | 0.7 | 0.7 |
| Re [mm] | 1 | 1 | 1 | 1 | 1 | 1 | 1 | 1 | 1 | 1 |
| A1 [deg] | 50 | 50 | 50 | 50 | 50 | 50 | 50 | 50 | 50 | 50 |
| A2 [deg] | 40 | 40 | 40 | 40 | 40 | 40 | 40 | 40 | 40 | 40 |

(continued)

| NUMERICAL VALUE EXAMPLE | | | | | | | | | | |
|---|---|---|---|---|---|---|---|---|---|---|
| EXAMPLE | 15 | 16 | 17 | 18 | 19 | 20 | 21 | 22 | 23 | 24 |
| A3 [deg] | 63 | 63 | 63 | 63 | 63 | 63 | 63 | 63 | 63 | 63 |
| Ae [deg] | 50 | 50 | 50 | 50 | 50 | 50 | 50 | 50 | 50 | 50 |
| D1 [mm] | 0.1 | 0.1 | 0.1 | 0.1 | 0.1 | 0.1 | 0.1 | 0.1 | 0.1 | 0.1 |
| D2 [mm] | 0.3 | 0.3 | 0.3 | 0.3 | 0.3 | 0.3 | 0.3 | 0.3 | 0.3 | 0.3 |
| Z/D2 | 0.43 | 0.37 | 0.30 | 0.23 | 0.17 | 0.10 | 0.03 | -0.03 | -0.10 | -0.17 |
| COLOR UNEVENNESS EVALUATION VALUE | 0.24 | 0.23 | 0.21 | 0.27 | 0.26 | 0.36 | 0.37 | 0.49 | 0.48 | 0.49 |

(Table 2C)

[Table 2C]

| NUMERICAL VALUE EXAMPLE | | | | | | | | | | |
|---|---|---|---|---|---|---|---|---|---|---|
| EXAMPLE | 25 | 26 | 27 | 28 | 29 | 30 | 31 | 32 | 33 | 34 |
| Y1 [mm] | 2.6 | 2.6 | 2.6 | 2.6 | 2.6 | 2.6 | 2.6 | 2.6 | 2.6 | 2.6 |
| Y2 [mm] | 2.4 | 2.4 | 2.4 | 2.4 | 2.4 | 2.4 | 2.4 | 2.4 | 2.4 | 2.4 |
| Y3 [mm] | 2.2 | 2.2 | 2.2 | 2.2 | 2.2 | 2.2 | 2.2 | 2.2 | 2.2 | 2.2 |
| Z1 [mm] | 2 | 2 | 2 | 2 | 2 | 2 | 2 | 2 | 2 | 2 |
| Z2 [mm] | 0.28 | 0.26 | 0.24 | 0.22 | 0.2 | 0.18 | 0.16 | 0.14 | 0.12 | 0.1 |
| Z3 [mm] | 0.4 | 0.4 | 0.4 | 0.4 | 0.4 | 0.4 | 0.4 | 0.4 | 0.4 | 0.4 |
| Z4 [mm] | 0.15 | 0.15 | 0.15 | 0.15 | 0.15 | 0.15 | 0.15 | 0.15 | 0.15 | 0.15 |
| R1 [mm] | 0.6 | 0.6 | 0.6 | 0.6 | 0.6 | 0.6 | 0.6 | 0.6 | 0.6 | 0.6 |
| R2 [mm] | 0.6 | 0.6 | 0.6 | 0.6 | 0.6 | 0.6 | 0.6 | 0.6 | 0.6 | 0.6 |
| R3 [mm] | 0.7 | 0.7 | 0.7 | 0.7 | 0.7 | 0.7 | 0.7 | 0.7 | 0.7 | 0.7 |
| Re [mm] | 1 | 1 | 1 | 1 | 1 | 1 | 1 | 1 | 1 | 1 |
| A1 [deg] | 50 | 50 | 50 | 50 | 50 | 50 | 50 | 50 | 50 | 50 |
| A2 [deg] | 40 | 40 | 40 | 40 | 40 | 40 | 40 | 40 | 40 | 40 |
| A3 [deg] | 63 | 63 | 63 | 63 | 63 | 63 | 63 | 63 | 63 | 63 |
| Ae [deg] | 50 | 50 | 50 | 50 | 50 | 50 | 50 | 50 | 50 | 50 |
| D1 [mm] | 0.1 | 0.1 | 0.1 | 0.1 | 0.1 | 0.1 | 0.1 | 0.1 | 0.1 | 0.1 |
| D2 [mm] | 0.3 | 0.3 | 0.3 | 0.3 | 0.3 | 0.3 | 0.3 | 0.3 | 0.3 | 0.3 |
| Z/D2 | 0.43 | 0.37 | 0.30 | 0.23 | 0.17 | 0.10 | 0.03 | -0.03 | -0.10 | -0.17 |
| COLOR UNEVENNESS EVALUATION VALUE | 0.23 | 0.29 | 0.21 | 0.22 | 0.30 | 0.36 | 0.40 | 0.39 | 0.42 | 0.51 |

(Table 2D)

[Table 2D]

| NUMERICAL VALUE EXAMPLE | | | | | | | | | | |
|---|---|---|---|---|---|---|---|---|---|---|
| EXAMPLE | 35 | 36 | 37 | 38 | 39 | 40 | 41 | 42 | 43 | 44 |
| Y1 [mm] | 2.6 | 2.6 | 2.6 | 2.6 | 2.6 | 2.6 | 2.6 | 2.6 | 2.6 | 2.6 |
| Y2 [mm] | 2.4 | 2.4 | 2.4 | 2.4 | 2.4 | 2.4 | 2.4 | 2.4 | 2.4 | 2.4 |
| Y3 [mm] | 2.2 | 2.2 | 2.2 | 2.2 | 2.2 | 2.2 | 2.2 | 2.2 | 2.2 | 2.2 |
| Z1 [mm] | 2 | 2 | 2 | 2 | 2 | 2 | 2 | 2 | 2 | 2 |
| Z2 [mm] | 0.28 | 0.26 | 0.24 | 0.22 | 0.2 | 0.18 | 0.16 | 0.14 | 0.12 | 0.1 |
| Z3 [mm] | 0.4 | 0.4 | 0.4 | 0.4 | 0.4 | 0.4 | 0.4 | 0.4 | 0.4 | 0.4 |
| Z4 [mm] | 0.15 | 0.15 | 0.15 | 0.15 | 0.15 | 0.15 | 0.15 | 0.15 | 0.15 | 0.15 |
| R1 [mm] | 0.65 | 0.65 | 0.65 | 0.65 | 0.65 | 0.65 | 0.65 | 0.65 | 0.65 | 0.65 |
| R2 [mm] | 0.6 | 0.6 | 0.6 | 0.6 | 0.6 | 0.6 | 0.6 | 0.6 | 0.6 | 0.6 |
| R3 [mm] | 0.6 | 0.6 | 0.6 | 0.6 | 0.6 | 0.6 | 0.6 | 0.6 | 0.6 | 0.6 |
| Re [mm] | 1 | 1 | 1 | 1 | 1 | 1 | 1 | 1 | 1 | 1 |
| A1 [deg] | 50 | 50 | 50 | 50 | 50 | 50 | 50 | 50 | 50 | 50 |
| A2 [deg] | 40 | 40 | 40 | 40 | 40 | 40 | 40 | 40 | 40 | 40 |
| A3 [deg] | 63 | 63 | 63 | 63 | 63 | 63 | 63 | 63 | 63 | 63 |
| Ae [deg] | 50 | 50 | 50 | 50 | 50 | 50 | 50 | 50 | 50 | 50 |
| D1 [mm] | 0.1 | 0.1 | 0.1 | 0.1 | 0.1 | 0.1 | 0.1 | 0.1 | 0.1 | 0.1 |
| D2 [mm] | 0.3 | 0.3 | 0.3 | 0.3 | 0.3 | 0.3 | 0.3 | 0.3 | 0.3 | 0.3 |
| Z/D2 | 0.43 | 0.37 | 0.30 | 0.23 | 0.17 | 0.10 | 0.03 | -0.03 | -0.10 | -0.17 |
| COLOR UNEVENNESS EVALUATION VALUE | 0.25 | 0.30 | 0.25 | 0.26 | 0.30 | 0.37 | 0.39 | 0.48 | 0.41 | 0.38 |

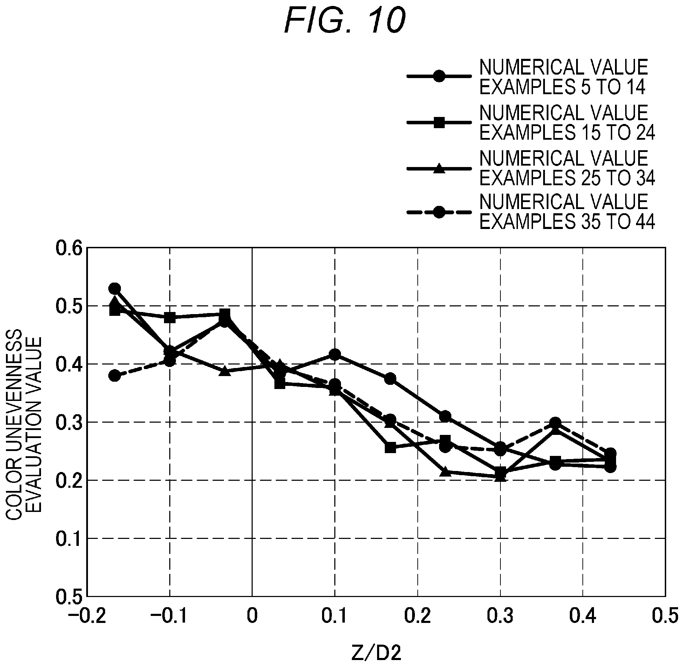

Fig. 10 is a graph showing the relationship between the color unevenness evaluation value and Formula (2) in Numerical Value Examples 5 to 44. In Fig. 10, the vertical axis represents the color unevenness evaluation value, and the horizontal axis represents the value of Z/D2.

As illustrated in Fig. 10, when the value of Z/D2 is 0.1 or more, the color unevenness evaluation value is 0.4 or less in most of the Numerical Value Examples. That is, it is found that the color unevenness of the illumination light can be favorably suppressed when Formula (2) is satisfied.

Tables 3A to 3B show parameter values of the endoscope illumination device according to Numerical Value Examples 45 to 56. In any of Numerical Value Examples 45 to 56, the value of D2/D1 is 3.0.

(Table 3A)

[Table 3A]

| NUMERICAL VALUE EXAMPLE | | | | | | | |
|---|---|---|---|---|---|---|---|
| EXAMPLE | 45 | 46 | 47 | 48 | 49 | 50 | 51 |
| Y1 [mm] | 2.6 | 2.6 | 2.6 | 2.6 | 2.6 | 2.6 | 2.6 |
| Y2 [mm] | 2.4 | 2.4 | 2.4 | 2.4 | 2.4 | 2.4 | 2.4 |
| Y3 [mm] | 2.2 | 2.2 | 2.2 | 2.2 | 2.2 | 2.2 | 2.2 |
| Z1 [mm] | 2 | 2 | 2 | 2 | 2 | 2 | 2 |
| Z2 [mm] | 0.22 | 0.22 | 0.22 | 0.22 | 0.22 | 0.22 | 0.22 |

(continued)

| NUMERICAL VALUE EXAMPLE | | | | | | | |
|---|---|---|---|---|---|---|---|
| EXAMPLE | 45 | 46 | 47 | 48 | 49 | 50 | 51 |
| Z3 [mm] | 0.4 | 0.4 | 0.4 | 0.4 | 0.4 | 0.4 | 0.4 |
| Z4 [mm] | 0.15 | 0.15 | 0.15 | 0.15 | 0.15 | 0.15 | 0.15 |
| R1 [mm] | 1 | 1 | 1 | 1 | 1 | 1 | 1 |
| R2 [mm] | 0.55 | 0.55 | 0.55 | 0.55 | 0.55 | 0.55 | 0.55 |
| R3 [mm] | 0.55 | 0.55 | 0.55 | 0.55 | 0.55 | 0.55 | 0.55 |
| Re [mm] | 0.5 | 0.8 | 1 | 1.2 | 1.4 | 1.6 | 1.8 |
| A1 [deg] | 15 | 15 | 15 | 15 | 15 | 15 | 15 |
| A2 [deg] | 75 | 75 | 75 | 75 | 75 | 75 | 75 |
| A3 [deg] | 80 | 80 | 80 | 80 | 80 | 80 | 80 |
| Ae [deg] | 50 | 50 | 50 | 50 | 50 | 50 | 50 |
| D1 [mm] | 0.1 | 0.1 | 0.1 | 0.1 | 0.1 | 0.1 | 0.1 |
| D2 [mm] | 0.3 | 0.3 | 0.3 | 0.3 | 0.3 | 0.3 | 0.3 |
| Re/R1 | 0.50 | 0.80 | 1.00 | 1.20 | 1.40 | 1.60 | 1.80 |
| COLOR UNEVENNESS EVALUATION VALUE | 0.23 | 0.23 | 0.23 | 0.21 | 0.27 | 0.36 | 0.37 |

(Table 3B)

[Table 3B]

| NUMERICAL VALUE EXAMPLE | | | | | |
|---|---|---|---|---|---|
| EXAMPLE | 52 | 53 | 54 | 55 | 56 |
| Y1 [mm] | 2.6 | 2.6 | 2.6 | 2.6 | 2.6 |
| Y2 [mm] | 2.4 | 2.4 | 2.4 | 2.4 | 2.4 |
| Y3 [mm] | 2.2 | 2.2 | 2.2 | 2.2 | 2.2 |
| Z1 [mm] | 2 | 2 | 2 | 2 | 2 |
| Z2 [mm] | 0.12 | 0.12 | 0.12 | 0.12 | 0.12 |
| Z3 [mm] | 0.4 | 0.4 | 0.4 | 0.4 | 0.4 |
| Z4 [mm] | 0.15 | 0.15 | 0.15 | 0.15 | 0.15 |
| R1 [mm] | 1.3 | 1.3 | 1.3 | 1.3 | 1.3 |
| R2 [mm] | 0.55 | 0.55 | 0.55 | 0.55 | 0.55 |
| R3 [mm] | 0.55 | 0.55 | 0.55 | 0.55 | 0.55 |
| Re [mm] | 1.2 | 1.4 | 1.6 | 1.8 | 2 |
| A1 [deg] | 15 | 15 | 15 | 15 | 15 |
| A2 [deg] | 75 | 75 | 75 | 75 | 75 |
| A3 [deg] | 80 | 80 | 80 | 80 | 80 |
| Ae [deg] | 50 | 50 | 50 | 50 | 50 |
| D1 [mm] | 0.1 | 0.1 | 0.1 | 0.1 | 0.1 |
| D2 [mm] | 0.3 | 0.3 | 0.3 | 0.3 | 0.3 |
| Re/R1 | 0.92 | 1.08 | 1.23 | 1.38 | 1.54 |

(continued)

| NUMERICAL VALUE EXAMPLE | | | | | |
|---|---|---|---|---|---|
| EXAMPLE | 52 | 53 | 54 | 55 | 56 |
| COLOR UNEVENNESS EVALUATION VALUE | 0.23 | 0.18 | 0.21 | 0.26 | 0.27 |

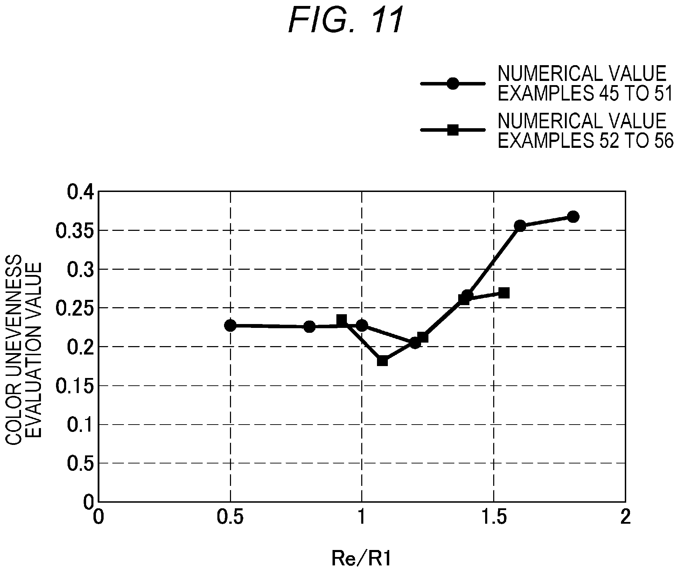

Fig. 11 is a graph showing the relationship between the color unevenness evaluation value and Formula (3) in Numerical Value Examples 45 to 56. In Fig. 11, the vertical axis represents the color unevenness evaluation value, and the horizontal axis represents the value of Re/R1.

As shown in Fig. 11, when the value of Re/R1 is 1.6 or less, the color unevenness evaluation value is 0.3 or less. That is, it is found that the color unevenness of the illumination light can be favorably suppressed when Formula (3) is satisfied.

Exemplary embodiments of the present invention have been described above. Embodiments of the present invention are not limited to the embodiments described above, and thus various modifications can be made without departing from the scope of the technical idea of the present invention. For example, any appropriate combination of the exemplary embodiments herein and conceivable embodiments is to be included in embodiments of the present invention.

**Claims**

1. An endoscope illumination device comprising:

   a light source unit that includes a light emitting element that emits excitation light and a phosphor that is excited by the excitation light to emit fluorescence, and emits illumination light in which the excitation light and the fluorescence are mixed; and
   a light distribution lens having an emission surface that emits the illumination light incident from the light source unit, wherein
   the emission surface includes a first emission surface and a second emission surface formed in different regions in the emission surface, and
   the thickness of the light emitting element and the thickness of the phosphor are defined such that the excitation light and the fluorescence emitted from the light source unit at the same angle and incident on the light distribution lens are emitted in substantially the same direction from the first emission surface or the second emission surface, respectively.

2. The endoscope illumination device according to claim 1, further comprising a support having an installation surface on which the light source unit is installed, wherein

   the light source unit is configured such that the phosphor covers the light emitting element,
   a thickness of the light emitting element is a thickness from a first surface of the light emitting element, the first surface being in contact with the installation surface, to a second surface of the light emitting element, the second surface being located on a side opposite to the first surface of the light emitting element and facing the light distribution lens,
   a thickness of the phosphor is a thickness from a first surface of the phosphor, the first surface being in contact with the installation surface, to a second surface of the phosphor, the second surface being located on a side opposite to the first surface of the phosphor and facing the light distribution lens, and
   when a thickness of the light emitting element is D1 and a thickness of the phosphor is D2, the following formula is satisfied:

   $$D2/D1 \leq 3.5.$$

3. The endoscope illumination device according to claim 2, wherein

   when a direction in which the support and the light source unit are arranged in order is a front direction, and a direction orthogonal to the front direction is a lateral direction,
   the first emission surface is positioned in the front direction of the light source unit,
   the second emission surface is positioned in the lateral direction of the light source unit, and
   the emission surface includes a connection surface connecting the first emission surface and the second emission surface.

4. The endoscope illumination device according to claim 3, wherein
the connection surface is formed of a curved surface.

5. The endoscope illumination device according to claim 3, wherein,

when a direction opposite to the front direction is a rear direction,
the second emission surface is formed to extend to the rear direction of the light source unit.

6. The endoscope illumination device according to claim 4, wherein

the light distribution lens
includes an incident surface facing the second surface,
emits the illumination light incident on the incident surface from the emission surface, and
includes a concave curved surface formed on the incident surface for increasing a light distribution angle of the illumination light.

7. The endoscope illumination device according to claim 6, wherein
the concave curved surface includes a plurality of curved surfaces having different curvature radii.

8. The endoscope illumination device according to claim 7, wherein,
when a distance in the front direction between a curvature center of a first curved surface formed closest to the connection surface among the plurality of curved surfaces and a center position of the light source unit is Z, the following formula is satisfied:

$$Z/D2 \geq 0.1.$$

9. The endoscope illumination device according to claim 8, wherein
the first curved surface is formed at a position intersecting a line segment connecting a center position of the curved surface forming the connection surface and the center position of the light source unit.

10. The endoscope illumination device according to claim 8, wherein

the light distribution lens is formed in an annular shape, and when a curvature radius of the curved surface forming the connection surface is Re and a curvature radius of the first curved surface is R1,
in at least a part of an angle range of the light distribution lens formed in the annular shape, the following formula is satisfied:

$$Re/R1 \leq 1.6.$$

11. The endoscope illumination device according to claim 10, wherein
the curved surface forming the connection surface is positioned in the front direction of the light source unit in the at least a part of the angle range.

12. The endoscope illumination device according to claim 1, wherein
a light distribution angle of the illumination light is 180° or greater.

13. The endoscope illumination device according to claim 1, wherein
the illumination light is white light.

14. An endoscope comprising:

an insertion tube;
an imaging unit provided at a distal end portion of the insertion tube; and
the endoscope illumination device according to any one of claims 1 to 13, provided around the imaging unit, wherein

the light distribution lens is formed in an annular shape so as to surround the imaging unit, and a plurality of the light source units are arranged around the imaging unit at intervals.

## FIG. 1

# FIG. 2A

# FIG. 2B

# FIG. 3

22

25

26a(26)

27

28a

28

26b(26)

6

Z

Y

X

# FIG. 4

FIG. 5

EP 4 544 984 A1

## FIG. 6

# FIG. 7

Y-axis: INDEX VALUE I (0.5 to 1.2)
X-axis: LIGHT DISTRIBUTION ANGLE [°] (0 to 120)

Labels: PVa, PVb

# FIG. 8A

Labels: 26, 126c, 27

Axes: Z, Y, X

## FIG. 8B

## FIG. 9

## FIG. 10

# FIG. 11

# *FIG. 12*

## FIG. 13A

## FIG. 13B

# FIG. 13C

## FIG. 14

EP 4 544 984 A1

## INTERNATIONAL SEARCH REPORT

| International application No. |
| --- |
| **PCT/JP2023/016623** |

| A. | CLASSIFICATION OF SUBJECT MATTER |
| --- | --- |

*A61B 1/07*(2006.01)i; *A61B 1/06*(2006.01)i
FI: A61B1/07 736; A61B1/06 531; A61B1/07 733

According to International Patent Classification (IPC) or to both national classification and IPC

| B. | FIELDS SEARCHED |
| --- | --- |

Minimum documentation searched (classification system followed by classification symbols)

A61B1/07; A61B1/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2013-175 A (FUJIFILM CORPORATION) 07 January 2013 (2013-01-07)<br>entire text, all drawings | 1-14 |
| A | WO 2014/054290 A1 (KABUSHIKI KAISHA TOSHIBA) 10 April 2014 (2014-04-10)<br>entire text, all drawings | 1-14 |
| A | JP 2013-215309 A (OLYMPUS CORPORATION) 24 October 2013 (2013-10-24)<br>entire text, all drawings | 1-14 |
| A | JP 2009-297313 A (FUJIFILM CORPORATION) 24 December 2009 (2009-12-24)<br>entire text, all drawings | 1-14 |
| A | WO 2021/152382 A1 (HOYA CORPORATION) 05 August 2021 (2021-08-05)<br>entire text, all drawings | 1-14 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **06 June 2023** | **20 June 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/016623**

| Patent document cited in search report | | | Publication date (day/month/year) | Patent family member(s) | | | Publication date (day/month/year) |
|---|---|---|---|---|---|---|---|
| JP | 2013-175 | A | 07 January 2013 | (Family: none) | | | |
| WO | 2014/054290 | A1 | 10 April 2014 | EP | 2905818 | A1 | |
| | | | | entire text, all drawings | | | |
| | | | | CN | 104718633 | A | |
| JP | 2013-215309 | A | 24 October 2013 | US | 2015/0038787 | A1 | |
| | | | | entire text, all drawings | | | |
| | | | | EP | 2835093 | A1 | |
| JP | 2009-297313 | A | 24 December 2009 | US | 2009/0306478 | A1 | |
| | | | | entire text, all drawings | | | |
| | | | | EP | 2130484 | A1 | |
| WO | 2021/152382 | A1 | 05 August 2021 | CN | 115151180 | A | |
| | | | | entire text, all drawings | | | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019136248 A